# EUROPÄISCHE PATENTSCHRIFT

(11) **EP 1 138 322 B1**
(45) Veröffentlichungstag und Bekanntmachung des Hinweises auf die Patenterteilung: **23.06.2004**
(21) Anmeldenummer: 01105544.9
(22) Anmeldetag: 06.03.2001
(51) Int. Cl.: A61K 9/48, C08F 283/06, C08F 8/12

(54) **Hartkapseln, enthaltend Polymerisate von Vinylestern und Polyethern, deren Verwendung und Herstellung**
Capsules comprising polymers of vinylesters and polyethers, their use and their preparation
Capsules contenant des polymères d' esters vinyliques et de polyéthers, leur utilisation et leur préparation

(30) Priorität: 29.03.2000 DE 10015468
(43) Veröffentlichungstag der Anmeldung: 04.10.2001
(73) Patentinhaber: BASF AKTIENGESELLSCHAFT, 67056 Ludwigshafen (DE)
(72) Erfinder: Angel, Maximilian, Dr., 67105 Schifferstadt (DE); Kolter, Karl, Dr., 67117 Limburgerhof (DE); Sanner, Axel, Dr., 67227 Frankenthal (DE); Gotsche, Michael, Dr., 68167 Mannheim (DE)

(56) Entgegenhaltungen:
- EP-A- 1 136 070
- WO-A-00/18375
- DE-A- 2 363 853

## Beschreibung

Die vorliegende Erfindung betrifft Hartkapseln, z.B. für pharmazeutische Anwendungen enthaltend Polymerisate, hergestellt durch Polymerisation von Vinylestern und gegebenenfalls eines weiteren radikalisch copolymerisierbaren Monomeren in Gegenwart einer polyetherhaltigen Verbindung, und anschließende zumindest teilweise Verseifung, enthaltend strukturverbessernde Hilfsstoffe sowie weitere übliche Hüllbestandteile, deren Verwendung und Herstellung.

Hartkapseln zeichnen sich dadurch aus, daß die Kapseln als zweiteilige, zusammengesteckte Leerkapseln produziert werden, die erst nach der Produktion gefüllt und verschlossen werden. Die Hartkapseln werden in den überwiegenden Fällen aus wäßriger Lösung im sogenannten Tauchverfahren (S. Stegmann, PZ Prisma, 5, 42-56, 1998) hergestellt. Eine Übersicht zum Stand der Technik des Spritzgießens zur Herstellung von pharmazeutischen Hartkapseln aus Stärke oder Gelatine ist von L. Eith et al. in Drug Dev. Ind. Pharm., 12, 2113-2126 (1986) gegeben. Bei näherer Betrachtung dieser Verarbeitung ist klar, daß die beiden Kapselteile mechanisch sehr stabil sein müssen, zumal die Füllmaschinen sehr schnell laufen und Formveränderungen sich sehr störend auf den Füllprozeß auswirken würden.

Da die beiden Formteile nach Befüllen dicht zusammengefügt werden, ist notwendig, daß das Kapselmaterial eine ausreichende Formstabilität aufweist.

Außerdem werden Hartkapseln für medizinische Anwendungen häufig zur Erhöhung der Lagerstabilität in sogenannten Blisterverpackungen verpackt. Beim Herausdrücken der Hartkapseln aus diesen Verpackungen findet eine mechanische Belastung statt, die nicht zu einem Verformen der Hartkapseln führen darf. Notwendigerweise müssen Hartkapseln deshalb eine ausreichende mechanische Stabilität aufweisen.

Bisher werden Hartkapseln für pharmazeutische Darreichungsformen überwiegend aus Gelatine hergestellt. Gelatine weist jedoch einige entscheidende Nachteile auf. So ist Gelatine ein Material tierischen Ursprungs und damit nicht kosher. Außerdem bleibt immer ein geringes Restrisiko von BSE, da zu ihrer Herstellung bevorzugt Gelatine von Rindern verwendet wird. Die Gewinnung einer geeigneten Gelatine ist sehr aufwendig und erfordert eine strenge Überwachung des Prozesses. Trotzdem sind die Chargenunterschiede aufgrund des tierischen Ursprungs, der einer gewissen Variabilität unterliegt, groß. Gelatine ist mikrobiell sehr anfällig, da sie einen guten Nährboden für Mikroorganismen darstellt. Bei der Herstellung, wie auch der Verwendung von solchen Verpackungsmaterialien müssen deshalb entsprechende Maßnahmen ergriffen werden. Häufig ist der Einsatz von Konservierungsmitteln unerläßlich.

Da Gelatine an sich ein sehr sprödes, wenig flexibles Material ist, muß es entsprechend weichgemacht werden, das heißt es müssen Weichmacher in Form von niedermolekularen Verbindungen zugesetzt werden. Diese erforderlichen Weichmacher treten häufig von der Hülle in das Füllgut über und führen dort zu Veränderungen. Die Hülle verarmt an Weichmachern und wird im Laufe der Lagerung spröde und mechanisch instabil.

Die Lösungsgeschwindigkeit von Gelatine ist verhältnismäßig langsam. Für schnelle Wirkstofffreisetzungen wäre eine höhere Auflösungsgeschwindigkeit in Magen- bzw. Darmsaft wünschenswert.

Zahlreiche Stoffe führen mit Gelatine zu Interaktionen wie z.B. Aldehyde, Polyphenole, reduzierende Zucker, mehrwertige Kationen, Elektrolyte, kationische oder anionische Polymere etc., wobei häufig Vernetzung eintritt und die Kapsel nicht mehr oder nur noch ganz langsam zerfällt bzw. sich auflöst. Für ein Arzneimittel sind solche Veränderungen verheerend, da die Wirksamkeit nicht mehr gegeben ist. Auch viele Arzneistoffe führen mit Gelatine zu Interaktionen. Zum Teil bilden sich während der Lagerung Abbauprodukte von Arzneistoffen mit beispielsweise aldehydischer Struktur, die zu einer Vernetzung der Gelatine führen. Da Gelatine sowohl saure wie auch basische Gruppen aufweist, ist verständlich, daß Reaktionen mit anderen geladenen Molekülen leicht eintreten.

Gelatine kann enzymatisch gespalten werden. Verunreinigungen durch Enzyme bzw. von Bakterien abgesonderte Enzyme können die Eigenschaften von Gelatine dramatisch verändern.

Aufgrund dieser vielen Nachteile hat es nicht an Versuchen gefehlt, die Gelatine in Hartkapseln ganz oder teilweise zu ersetzen.

Es sind deshalb Versuche gemacht worden, synthetische Polymerisate zu finden, die für die Herstellung von Hartkapseln eingesetzt werden können.

Polyvinylalkohol ist beispielsweise für diesen Zweck beschrieben. Polyvinylalkohol weist jedoch eine langsame Lösungsgeschwindigkeit auf, erfordert ebenfalls zusätzliche Weichmacher, die wiederum migrieren können und die, wie oben bereits beschrieben, die Eigenschaften des Füllguts verändern können, und kann außerdem in Folge innerer Kristallisation stark verspröden. Insbesondere bei niedriger Umgebungsfeuchte nimmt die Flexibilität im Laufe der Lagerung dramatisch ab.

In der JP Sho-45-1277 wurde beispielsweise Polyvinylalkohol als Grundstoff von Hartkapseln verwendet. In der DE OS-1 965 584 ist ebenfalls die Verwendung von Polyvinylalkohol und anderen Polymeren für die medizinischen Hartkapseln beschrieben.

Diese Polymerisate erwiesen sich in Technik und Praxis der Kapselherstellung und ihrer Anwendung nicht als vollwertiger Ersatz für gebräuchliche Gelatine.

DE-OS 23 63 853 beschreibt selbsttragende Packungen oder Kapseln für Medikamente, die unter Verwendung von Pfropfcopolymerisaten von Polyvinylalkohol auf Polyethylenglykol hergestellt werden. Die für diese Anwendung bevorzugten Pfropfpolymerisate werden durch Pfropfung von Vinylacetat auf Polyethylenglykol mit einem Molekulargewicht von 20000 bis 25000 und anschließender Methanolyse der Vinylacetat-Einheiten hergestellt. Solche Polymerisate sind sehr weich und leicht verformbar.

Pfropfpolymerisate von Polyvinylalkohol auf Polyalkylenglykole sind bereits anderen Patentschriften beschrieben.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Die Verwendung der beschriebenen Pfropfpolymerisate für Hartkapseln ist nicht offenbart.

Die bisher im Stand der Technik beschriebenen Polymerisate weisen aufgrund ihrer Elastizität und Flexibilität zur Herstellung von Hartkapseln unzureichende Eigenschaften auf.

Aufgabe der Erfindung war es daher, synthetische Polymerisate mit größerer Formstabilität zur Herstellung von Hartkapseln für pharmazeutische Darreichungsformen zu finden, die der Gelatine und vielen bekannten Ersatzmaterialien überlegen sind.

Die Aufgabe wurde erfindungsgemäß gelöst durch Hartkapseln, enthaltend
(A) Polymerisate, die erhältlich sind durch radikalische Polymerisation von
   a) mindestens einem Vinylester in Gegenwart von
   b) polyetherhaltigen Verbindungen und
   c) gegebenenfalls eines oder mehreren weiteren copolymerisierbaren Monomeren und anschließender zumindest teilweiser Verseifung der Esterfunktionen der ursprünglichen Monomere a),
   mit der Maßgabe, daß bei Abwesenheit eines weiteren copolymerisierbaren Monomers c), die polyetherhaltige Verbindung b) ein mittleres Molekulargewicht ≤ 10 000 (Zahlenmittel) haben muß.
(B) gegebenenfalls strukturverbessernde Hilfsstoffe und
(C) weitere übliche Hüllbestandteile.

Überraschenderweise wurde dabei gefunden, daß insbesondere die Hinzunahme eines weiteren Comonomeren c) bei der Polymerisation zu härteren Polymerisaten führt, die für die Herstellung von Hartkapseln mit verbesserter Formstabilität geeignet sind.

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften polyetherhaltigen Verbindungen und Homo- oder Copolymerisaten der Monomeren a) und c) zu verstehen.

Als polyetherhaltige Verbindungen (b) können sowohl Polyalkylenoxide auf Basis von Ethylenoxid, Propylenoxid, Butylenoxid und weiteren Alkylenoxiden als auch Polyglycerin verwendet werden. Je nach Art der Monomerbausteine enthalten die Polymere folgende Struktureinheiten.

-(CH₂)₂-O-, -(CH₂)₃-O-, -(CH₂)₄-O-, -CH₂-CH(R⁶)-O-,

-CH₂-CHOR⁷-CH₂-O-

mit
- R⁶: C₁-C₂₄-Alkyl ;
- R⁷: Wasserstoff, C₁-C₂₄-Alkyl, R⁶-C(=0)-, R⁶-NH-C(=O)-.

Dabei kann es sich bei den Struktureinheiten sowohl um Homopolymere als auch um statistische Copolymere und Blockcopolymere handeln.

Bevorzugt werden als Polyether (b) Polymerisate der allgemeinen Formel I verwendet, 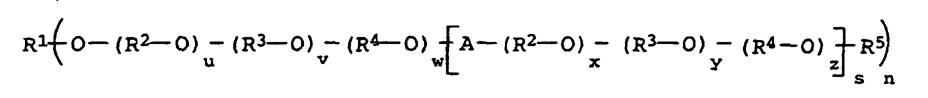 in der die Variablen unabhängig voneinander folgende Bedeutung haben:
- R¹: Wasserstoff, C₁-C₂₄-Alkyl, R⁶-C(=O)-, R⁶-NH-C(=O)-, Polyalkoholrest;
- R⁵: Wasserstoff, C₁-C₂₄-Alkyl, R⁶-C(=O)-, R⁶-NH-C(=O)-;
- R² bis R⁴: -(CH₂)₂-,- (CH₂)₃-,-(CH₂)₄-, -CH₂-CH(R⁶ ) -, -CH₂-CHOR⁷-CH₂-;
- R⁶: C₁-C₂₄-Alkyl;
- R⁷: Wasserstoff, C₁-C₂₄-Alkyl, R⁶-C(=O)-, R⁶-NH-C(=O)-;
- A: -C(=O)-O, -C(=O)-B-C(=O)-O,
-C(=O)-NH-B-NH-C(=O)-O;
- B: -(CH₂)ₜ-, Arylen, ggf. substituiert;
- n: 1 bis 1000;
- s: 0 bis 1000;
- t: 1 bis 12;
- u: 1 bis 5000;
- v: 0 bis 5000;
- w: 0 bis 5000:
- x: 0 bis 5000;
- y: 0 bis 5000;
- z: 0 bis 5000
mit der Maßgabe, daß bei Abwesenheit eines weiteren copolymerisierbaren Monomers c),
- n: 1 bis 200;
- s: 0 bis 200;
- t: 1 bis 12;
- u: 1 bis 250;
- v: 0 bis 250;
- w: 0 bis 250;
- x: 0 bis 250;
- y: 0 bis 250;
- z: 0 bis 250 bedeutet und das Molekulargewicht des Polyethers ≤ 10000 liegt.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden.

Als Alkylreste für R¹ und R⁵ bis R⁷ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich kleiner 1000000 (nach Zahlenmittel), bevorzugt im Bereich von 300 bis 100000, besonders bevorzugt im Bereich von 500 bis 50000, ganz besonders bevorzugt im Bereich von 800 bis 40000. Bei Abwesenheit eines weiteren copolymerisierbaren Monomeren c) liegt das mittlere Molekulargewicht (Zahlenmittel) der Polyether im Bereich kleiner gleich 10000 (Zahlenmittel), bevorzugt im Bereich von 300 bis 10000, besonders bevorzugt im Bereich von 500 bis 10000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Im folgenden beziehen sich die Zahlenwerte in Klammern auf den Fall, daß kein zusätzlich copolymerisierbares Monomer c) anwesend ist.

Besonders bevorzugt werden als Polyether (b) Polymerisate der allgemeinen Formel I mit einem mittleren Molekulargewicht von 300 bis 100.000 (10.000) (nach dem Zahlenmittel), in der die Variablen unabhängig voneinander folgende Bedeutung haben:
- R¹: Wasserstoff, C₁-C₁₂-Alkyl, R⁶-C(=O)-, R⁶-NH-C(=O)-, Polyalkoholrest;
- R⁵: Wasserstoff, C₁-C₁₂-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₁₂-Alkyl;
- R⁷: Wasserstoff, C₁-C₁₂-Alkyl, R⁶-C (=O) -, R⁶-NH-C (=O)-;
- n: 1 bis 8 (1 bis 8);
- s: 0 (0) ;
- u: 2 bis 2000 (250);
- v: 0 bis 2000 (250);
- w: 0 bis 2000 (250).

Ganz besonders bevorzugt werden als Polyether b) Polymerisate der allgemeinen Formel I mit einem mittleren Molekulargewicht von 500 bis 50000 (10.000)(nach dem Zahlenmittel), in der die Variablen unabhängig voneinander folgende Bedeutung haben:
- R¹: Wasserstoff, C₁-C₆-Alkyl, R⁶-C(=O)-, R⁶-NH-C(=O)-;
- R⁵: Wasserstoff, C₁-C₆-Alkyl, R⁶-C(=O)-, R⁶-NH-C(=O)-;
- R² bis R⁴: -(CH₂)₂-, -(CH₂)₃- -(CH₂)₄-, -CH₂-CH(R⁶)-, -CH₂-CHOR⁷-CH₂-;
- R⁶: C₁-C₆-Alkyl;
- R⁷: Wasserstoff, C₁-C₆-Alkyl, R⁶-C(=O)-, R⁶-NH-C(=O)-;
- n: 1 (1);
- s: 0 (0);
- u: 5 bis 500 (250):
- v: 0 bis 500 (250);
- w: 0 bis 500 (250).

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallyamide oder Polyalkylenoxidvinylamide verwendet werden. Selbstverständlich können auch Copolymerisate solcher Monomere mit anderen ethylenisch ungesättigten Monomeren eingesetzt werden. Für die Polymerisation in Gegenwart der Polyether b) seien als Komponente a) folgende radikalisch polymerisierbare Monomere genannt:

Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbonsäuren, insbesondere der C₁-C₆-Carbonsäuren, verwendet. Ganz besonders bevorzugt ist Vinylacetat.

Selbstverständlich können auch Mischungen der jeweiligen Monomeren aus der Gruppe a) copolymerisiert werden.

Zur Einstellung der gewünschten mechanischen Eigenschaften kann zusätzlich zum Vinylester (a) mindestens ein ethylenisch ungesättigtes copolymerisierbares Comonomeres (c) eingesetzt werden. Der Anteil dieser zusätzlichen Monomere liegt bevorzugt zwischen 0 und 50 Gew.-%, ganz besonders bevorzugt zwischen 0 und 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten eingesetzten ethylenisch ungesättigten Comonomere (c) können durch die folgende allgemeine Formel beschrieben werden:

X-C(O)CR¹⁵ = CHR¹⁴

wobei
X ausgewählt ist aus der Gruppe der Reste -OH, -OM, -OR¹⁶, NH₂, -NHR¹⁶, N(R¹⁶)₂;
M ist ein Kation ausgewählt aus der Gruppe bestehend aus: Na⁺, K⁺, Mg⁺⁺, Ca⁺⁺, Zn⁺⁺, Nah₄⁺, Alkylammonium, Dialkylammonium, Trialkylammonium und Tetraalkylammonium;
   die Reste R¹⁶ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.
R¹⁵ und R¹⁴ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III) 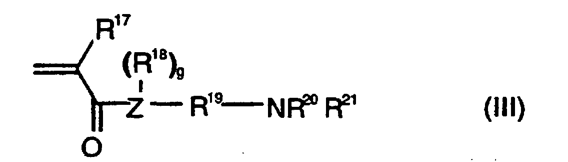 mit
- R¹⁷ =: H, Alkyl mit 1 bis 8 C-Atomen,
- R¹⁸=: H, Methyl,
- R¹⁹ =: Alkylen mit 1 bis 24 C-Atomen, optional substituiert durch Alkyl,
- R²⁰, R²¹ =: C₁-C₄₀ Alkylrest
- Z =: Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyloder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin R²² bis R²⁴ unabhängig voneinander für Wasserstoff, C₁-C₄-Alkyl oder Phenyl steht:

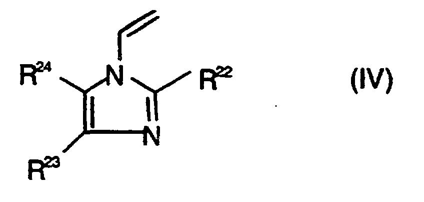

Weitere geeignete Comonomere (c) sind Diallylamine der allgemeinen Formel (V) 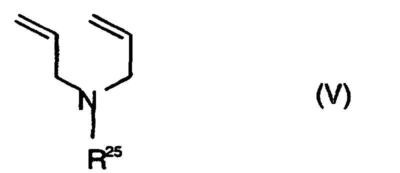 mit R²⁵ = C₁- bis C₂₄-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)-acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl (meth)-acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)-butyl]methacrylamid, N-[8-(dimethylamino)octyl]methacrylamid, N-[12-(dimethylamino)dodecyl]methacrylamid, N-[3-(diethylamino)-propyl]methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)-propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden (R²⁶ = C₁- bis C₄₀-Alkyl).

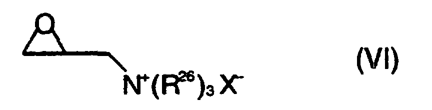

Beispiele hierfür sind zum Beispiel:

(Meth)acryloyloxyhydroxypropyltrimethylammoniumchlorid und (Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.

Bevorzugt werden silikonfreie Regler eingesetzt.

Die vorteilhaften mechanischen Eigenschaften können auch durch die Verwendung von vernetzenden Monomeren als Monomere c) erreicht werden. Der Begriff vernetzend bedeutet, daß die Monomere mindestens zwei ethylenisch ungesättigten Doppelbindungen besitzen, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B. Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyloder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0 bis 5 Gew.-%, ganz besonders bevorzugt 0 bis 2 Gew.-%.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azobis-(2-amidonopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden organische Peroxide eingesetzt.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die polyetherhaltige Verbindung b) in mindestens einem _{M}onomer der Gruppe a) und gegebenenfalls mindestens eines weiteren Comonomeren der Gruppe c) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung b), mindestens einem Monomeren der Gruppe a), gegebenenfalls mindestens eines weiteren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), gegebenenfalls mindestens eines weiteren Comonomeren der Gruppe c) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Polymerisation von
a) 10 bis 98 Gew.-% mindestens eines Vinylesters von C₁-C₂₄-Carbonsäuren in Gegenwart von
b) 2 bis 90 Gew.-% mindestens einer polyetherhaltigen Verbindung und
c) 0 bis 50 Gew.-% eines oder mehreren weiteren copolymerisierbaren Monomeren

Besonders bevorzugt sind Polymere, die erhältlich sind durch radikalische Polymerisation von
a) 50 bis 97 Gew.-% mindestens eines Vinylesters von C₁-C₂₄-Carbonsäuren in Gegenwart von
b) 3 bis 50 Gew.-% mindestens einer polyetherhaltigen Verbindung und
c) 0 bis 20 Gew.-% eines oder mehreren weiteren copolymerisierbaren Monomeren

Ganz besonders bevorzugt sind Polymere, die erhältlich sind durch radikalische Polymerisation von
a) 65 bis 97 Gew.% mindestens eines Vinylesters von C₁-C₂₄-Carbonsäuren in Gegenwart von
b) 3 bis 35 Gew.% mindestens einer polyetherhaltigen Verbindung und
c) 0 bis 20 Gew.% eines oder mehreren weiteren copolymerisierbaren Monomeren

Zur Herstellung der erfindungsgemäß verwendeten Polymeren werden die Estergruppen der ursprünglichen Monomere a) und gegebenenfalls weiterer Monomere nach der Polymerisation durch Hydrolyse, Alkoholyse oder Aminolyse gespalten. Im nachfolgenden wird dieser Verfahrensschritt allgemein als Verseifung bezeichnet. Die Verseifung erfolgt in an sich bekannter Weise durch Zugabe einer Base, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base, von der Verseifungstemperatur, der Verseifungszeit und dem Wassergehalt der Lösung.

Der Verseifungsgrad der Polyvinylestergruppen liegt im Bereich von 1 bis 100 %, bevorzugt im Bereich von 40 bis 100 %, besonders bevorzugt im Bereich von 65 bis 100 %, ganz besonders bevorzugt im Bereich von 80 bis 100 %.

Die so hergestellten Polymerisate können durch Umsetzung von im Polymer vorhandenen Hydroxyl- und/oder Aminofunktionen mit Epoxiden der Formel VI nachträglich kationisiert werden (R²⁶ = C₁ bis C₄₀ Alkyl) .

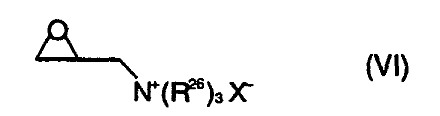

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden.

Die Epoxide der Formel VI können auch in situ durch Umsetzung der entsprechenden Chlorhydrine mit Basen, beispielsweise Natriumhydroxid, erzeugt werden.

Bevorzugt wird 2,3-Epoxypropyl-trimethylammoniumchlorid bzw. 3-Chlor-2-hydroxypropyl-trimethylammoniumchlorid eingesetzt.

Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25?C und Polymerkonzentrationen, die je nach K-Wert-Bereich zwischen 0,1 Gew.-% und 5 Gew.-% liegen.

Nach der Verseifung können die Polymerlösungen zur Entfernung von Lösungsmitteln wasserdampfdestilliert werden. Nach der Wasserdampfdestillation erhält man je nach Verseifungsgrad, Art der Polyether b), der Vinylester a) und der eventuell eingesetzten Monomere c) wäßrige Lösungen oder Dispersionen.

Die mechanischen Eigenschaften der Polymerisate zur Herstellung von Hartkapseln für pharmazeutische Darreichungsformen können jedoch auch durch nachträgliche Vernetzung positiv beeinflußt werden.

Gegenstand der Erfindung ist deshalb auch die Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen
c) gegebenenfalls eines oder mehreren copolymerisierbaren Monomeren
und anschließender zumindest teilweiser Verseifung der Esterfunktionen der ursprünglichen Monomeren a), zur Herstellung von Hartkapseln, dadurch gekennzeichnet, daß die erhaltenen Polymerisate nachträglich durch eine polymeranaloge Umsetzung vernetzt werden.

Die nachträgliche Vernetzung kann dabei erfolgen, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgraden wasserquellbare bzw. unlösliche Produkte.

Beispielsweise können die erfindungsgemäßen Polymerisate mit Dialdehyden und Diketonen, z.B. Glyoxal, Glutaraldehyd, Succindialdehyd oder Terephthalaldehyd, umgesetzt werden. Des weiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon.

Weiterhin eignen sich anorganische Verbindungen wie Borsäure oder Borsäuresalze, beispielsweise Natriummetaborat, Borax (Dinatriumtetraborat), sowie Salze mehrwertiger Kationen, z.B. Kupfer(II)-salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.

Der Anteil Borsäure bzw. Borsäuresalze bezogen auf die erfindungsgemäßen Polymere beträgt 0 bis 15 Gew-%, bevorzugt 0 bis 10 Gew.-%, besonders bevorzugt 0 bis 5 Gew.-%.

Die Lösungen und Dispersionen der erfindungsgemäßen Polymerisate können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Anstelle der wasserdampfdestillierten Polymerlösungen können auch die alkoholischen Polymerlösungen direkt in Pulverform überführt werden.

Die erfindungsgemäßen wasserlöslichen oder wasserdispergierbaren Polyalkylenoxid- bzw. Polyglycerin-haltigen Polymerisate eignen sich hervorragend zur Herstellung von Hartkapseln für pharmazeutische Darreichungsformen.

Die Polymerisate lassen sich mit den o.g. Verfahren mir hoher Reproduzierbarkeit herstellen. Zu ihrer Herstellung werden keine Materialien tierischen Ursprungs verwendet und da auch keine pflanzlichen Materialien eingesetzt werden, stellt sich das Problem von Produkten gentechnologischen Ursprungs nicht.

Mikrobiologisch sind die Polymerisate nicht besonders anfällig, weil sie keinen guten Nährboden für Mikroorganismen darstellen. Weder durch Enzyme noch durch Hydrolyse werden die Polymerketten abgebaut. Daher ist auch die Herstellung von Lösungen für die Filmherstellung und Verkapselung unproblematisch.

Typische verpackte Materialien sind bevorzugt pharmazeutische Erzeugnisse, wie feste und flüssige Wirkstoffe, aber auch Vitamine, Carotinoide, Mineralstoffe, Spurenelemente, Nahrungsergänzungsstoffe, Gewürze sowie Süßstoffe. Weiterhin können die Kapseln für kosmetische Wirkstoffe ("personal care"), wie beispielsweise Haar- und Hautformulierungen, für Öle, Duftstoffe, Badezusätze oder Proteine verwendet werden. Weitere Anwendungen im Bereich "personal care" sowie weitere Anwendungen für wasserlösliche Verpackungen sind in der WO 99/40156 genannt.

Weitere verpackte Materialien können sein, z.B. Reinigungsmittel, wie Seifen, Waschmittel, Farb- und Bleichmittel, Agrarchemikalien wie Düngemittel (-kombinationen), Pflanzenschutzmittel wie Herbizide, Fungizide oder Pestizide und Saatgut.

Bevorzugt wird in Hartkapseln festes Füllgut verpackt.

Generell lassen sich mit den erfindungsgemäßen Polymerisaten Inhaltsstoffe verpacken, die geschützt werden sollen, bevor sie in eine wässerige Umgebung gebracht werden.

Die Auflösungsgeschwindigkeit der erfindungsgemäßen Polymere und daraus hergestellter Hartkapseln ist enorm hoch und übertrifft die von Gelatine und Polyvinylalkohol deutlich. Außerdem sind die Polymere kaltwasserlöslich. Gelatine und Polyvinylalkohol lösen sich erst bei höheren Temperaturen. Da viele Arzneistoffe schnell nach der Einnahme wirken sollen, ist dieses Lösungsverhalten insbesondere für diese Verwendung ein klarer Vorteil.

Im Gegensatz zu Gelatine können in die erfindungsgemäßen Hüllen auch Stoffe verkapselt werden, die zu Interaktionen neigen, wie z.B. Aldehyde oder mehrwertige Kationen. Eine Verlängerung der Auflösungsgeschwindigkeit ist nicht zu erkennen.

Hartkapseln der erfindungsgemäßen Zusammensetzung lassen sich hervorragend unter Verwendung von wäßrigen Polymerlösungen oder Polymersuspensionen coaten. So kann durch Aufsprühen von Kollicoat MAE 30 DP (Methacrylsäure-Copolymer Typ C der USP) in einem Horizontaltrommelcoater ein stark auf der Oberfläche haftender magensaftresistenter Überzug aufgebracht werden, der zudem lagerungsstabil ist.

Zur Erzielung einer Magensaftresistenz können in der Hülle außerdem 20 bis 80 %, vorzugsweise 30 bis 70 % eines magensaftresistenten Polymers enthalten sein.

Den Polymerisaten können strukturverbessernde Hilfsstoffe zugesetzt werden, um die mechanischen Eigenschaften wie Flexibilität und Festigkeit zu modifizieren. Diese strukturverbessernden Hilfsstoffe lassen sich in 2 große Gruppen einteilen
A) Polymere mit einem Molekulargewicht größer 50000, vorzugsweise größer 100000
B) Stoffe die zu einer Vernetzung der Polymerketten der Polymeren führen, vorzugsweise Aldehyde, Borsäure und ihre Salze,
sowie gegebenenfalls Stoffe, die zu einer Vernetzung der Polymerketten der strukturverbessernden Hilfsstoffen führen, vorzugsweise Erdalkaliionen, Amine, Tannine sowie Aldehyde und Borate.

Als Polymere mit hohem Molekulargewicht können Stoffe aus folgenden Stoffklassen eingesetzt werden:

Polyaminosäuren, wie Gelatine, Zein, Sojaprotein sowie Derivate davon,

Polysaccharide wie Stärke, abgebaute Stärke, Maltodextrine, Carboxymethylstärke, -Cellulose, Hydroxypropylmethylcellulose, Hydroxypropylcellulose, Hydroxyethylcellulose, Methylcellulose, Carboxymethylcellulose, Ethylcellulose, Celluloseacetat, Celluloseacetatphthalat, Hydroxypropylcelluloseacetatphthalat, Hydroxypropylcelluloseacetatsuccinat, Hemicellulose, Galactomannane, Pectine, Alginate, Carrageenane, Xanthan, Gellan, Dextran, Curdlan, Pullulan, Chitin, sowie Derivate davon, synthetische Polymere wie Polyacrylsäure, Polymethacrylsäure, Copolymerisate aus Acrylsäure- und Methacrylsäureestern, Polyvinylalkohole, Polyvinylacetat, Polyethylenglykole, Polyoxyethylen-Polyoxypropylen-Blockpolymere, Polyvinylpyrrolidone sowie Derivate davon.

Diese Polymere mit hohem Molekulargewicht bilden ein Netzwerk mit den Polymeren und erhöhen so die Festigkeit der Hartkapseln. Die Flexibilität leidet, sofern keine sehr hohen Konzentrationen verwendet werden in aller Regel nicht. Überraschenderweise sind hierfür nicht nur wasserlösliche, sondern auch wasserunlösliche Polymere wie Copolymere aus Acrylsäure- und Methacrylsäureestern geeignet. Bleibt die Konzentration dieser wasserunlöslichen Polymere unter 50 %, zerfallen die Kapseln immer noch.

In ähnlicher Weise wirken Stoffe, die zu einer Vernetzung entweder der Polymerketten der Polymere oder der zugesetzten hochmolekularen Polymere führen.

Neben den genannten Komponenten können die erfindungsgemäßen Hartkapseln noch weitere übliche Bestandteile enthalten. Dazu zählen Füllstoffe, Formtrennmittel, Rieselhilfsmittel, Stabilisatoren sowie wasserlösliche oder wasserunlösliche Farbstoffe, Aromen und Süßstoffe.

Farbstoffe sind z.B. Eisenoxide, Titandioxid, die in einer Menge von etwa 0,001 bis 10, vorzugsweise von 0,5 bis 3 Gew.-% zugesetzt werden, Triphenylmethanfarbstoffe, Azofarbstoffe, Chinolinfarbstoffe, Indigofarbstoffe, Carotinoide, um die Kapseln einzufärben, Opakisierungsmittel wie Titandiodid oder Talkum, um die Lichtundurchlässigkeit zu erhöhen und um Farbstoffe einzusparen.

Aromen und Süßstoffe sind insbesondere dann von Bedeutung, wenn ein schlechter Geruch oder Geschmack überdeckt werden soll und die Kapsel zerbissen wird.

Konservierungsmittel sind in aller Regel nicht erforderlich.

Füllstoffe sind z.B. anorganische Füllstoffe wie Oxide von Magnesium, Aluminium, Silicium, Titan oder Calciumcarbonat. Der bevorzugte Konzentrationsbereich für die Füllstoffe ist etwa 1 bis 50 Gew.-%, besonders bevorzugt 2 bis 30 Gew.-% bezogen auf das Gesamtgewicht sämtlicher Komponenten.

Schmiermittel sind Stearate von Aluminium, Calcium, Magnesium und Zinn, sowie Magnesiumsilikat, Silikone und ähnliche. Der bevorzugte Konzentrationsbereich ist etwa 0,1 bis 5 Gew.-%, besonders bevorzugt etwa 0,1 bis 3 Gew.-% bezogen auf das Gesamtgewicht sämtlicher Komponenten.

Rieselhilfsmittel sind z.B. feinteilige bzw. feinstteilige Kieselsäuren, ggf. modifiziert. Der bevorzugte Konzentrationsbereich ist 0,05 bis 3 Gew.-%, besonders bevorzugt 0,1 bis 1 Gew.-% bezogen auf das Gesamtgewicht sämtlicher Komponenten.

Zur Verbesserung der thermoplastischen Eigenschaften der Massen kommen als Zusätze auch thermoplastische Polymere wie Polyethylen, Polypropylen, Polyisobutylen, Polystyrol, Polyacrylnitril, Polyvinylcarbazole, Polyacrylat, Polymethacrylat, Polyvinylchlorid, Polyvinylacetat, Polyamid, Polyester, Polyurethan, Polycarbonat, Polyalkylenterephthalat, sowie Copolymere aus Ethylen/Vinylacetat, Ethylen/Vinylalkohol, Ethylen/(Meth-) Acrylsäure, Ethylen/(Meth-) Acrylsäureester, ABS-Copolymer, SAN-Copolymere, Ethylen/Maleinsäureanhydrid-Copolymere in Frage. Das erfindungsgemäß als Ausgangsmaterial verwendete Pfropfpolymerisat kann mit unterschiedlichen bekannten Zusätzen wie Füllstoffen, Formtrennmitteln, Rieselhilfsmitteln, Plastifizierungsmitteln, Stabilisatoren und/oder Färbemitteln gemischt werden.

Ein Sonderfall stellt die Einarbeitung von Wirkstoffen in die Hülle dar. Dies kann vorteilhaft sein, um inkompatible Wirkstoffe voneinander zu trennen. Der Wirkstoff mit der geringsten Dosierung sollte dann in die Hülle eingearbeitet werden.

Die Hülle der erfindungsgemäßen Verpackungsmaterialien besteht aus 10 bis 100 %, vorzugsweise 20 bis 98 % Polymerisaten, gegebenenfalls 0 bis 80 %, vorzugsweise 1,9 bis 50 % strukturverbessernden Hilfsstoffen und 0,1 bis 30 % weiteren üblichen Bestandteilen.

Die folgenden Beispiele sollen die Erfindung näher erläutern, ohne sie jedoch darauf einzuschränken.

Herstellvorschrift für die Beispiele 1 bis 14 sowie für die Vergleichsbeispiele 15 bis 18

In einem Polymerisationsgefäß wird die polyetherhaltige Verbindung vorgelegt und unter Rühren und leichtem Stickstoffstrom auf 80° erhitzt. Unter Rühren werden Vinylacetat und gegebenenfalls das weitere Monomere in 3 h zudosiert. Gleichzeitig wird eine Lösung von 1,4 g tert.-Butylperoxy-2-ethylhexanoat in 30 g Methanol ebenfalls in 3 h zugegeben. Danach wird noch 2 h bei 80°C gerührt. Nach dem Abkühlen wird das Polymerisat in 450 ml Methanol gelöst. Zur Verseifung gibt man bei 30°C 50 ml einer 10%igen methanolischen Natriumhydroxidlösung zu. Nach ca. 40 min. wird die Reaktion durch Zugabe von 750 ml 1%iger Essigsäure abgebrochen. Das Methanol wird durch Destillation entfernt.

Die K-Werte wurden 1%ig in N-Methylpyrrolidon bestimmt.

Von den so hergestellten Polymerisaten werden Filme gegossen und bei 54 % relativer Luftfeuchtigkeit die Reißdehnung bestimmt. Anhand von einzeln durch Tauchen hergestellten Kapselteilen wurde die Formstabilität nach 4wöchiger Lagerung geprüft. Es zeigte sich dabei, daß die Reißdehnung sehr gut mit der Formstabilität korreliert. Bei hohen Reißdehnungen konnten aus den Polymerisaten keine formstabilen Kapseln hergestellt werden.

**Tabelle**

| Beispiel | Pfropfgrundlage (1) | Vinylester | Comonomer | K-Wert | Verseifungsgrad [%] | Reißdehnung [%] | Formstabilität der hergestellten Kapseln |
|---|---|---|---|---|---|---|---|
| 1 | PEG 6000, 72g | Vinylacetat, 396g | Methylmethaclylat, 12g | 47 | > 95 | 21 | Formstabil |
| 2 | PEG 20000 72 g | Vinylacetat, 328 g | N-Vinylpyrrolidon, 82 g | 61 | > 95 | 20 | Formstabil |
| 3 | PEG 20000, 72 g | Vinylacetat, 362 g | 3-Methyl-1-vinylimidazoliummethylsulfat, 48 g | 53 | > 95 | 40 | Formstabil |
| 4 | PEG 6000, 72g | Vinylacetat, 367g | N-Vinylfonnamid, 41g | 57 | > 95 | 35 | Formstabil |
| 5 | PEG 6000, 72 g | Vinylacetat, 326 g | N-Vinylformamid, 82 g | 67 | >95 | 25 | Formstabil |
| 6 | PEG 35000, 270 g | Vinylacetat, 410 g | Pentaerythrittriallylether, 1,6 g | 71 | 95 | 20 | Formstabil |
| 7 | PEG 35000, 270 g | Vinylacetat, 410 g | Pentaerythrittriallylether, 0,8 g | 65 | 94 | 40 | Formstabil |
| 8 | PEG 35000, 270 g | Vinylacetat, 410 g | N,N'-Divinylethylenharnstoff, 0,7 g | 73 | 95 | 42 | Formstabil |
| 9 | PEG 150072 g | Vinylacetat; 410 g | - | 47 | > 95 | 17 | Formstabil |
| 10 | PEG 400072 g | Vinylacetat, 410 g | - | 51 | >95 | 32 | Formstabil |
| 11 | PEG 6000, 72 g | Vinylacetat, 410 g | - | 54 | > 95 | 42 | Formstabil |
| 12 | PEG 6000, 137 g | Vinylacetat, 410 g | - | 49 | >95 | 46 | Formstabil |
| 13 | PEG 6000, 22 g | Vinylacetat, 410 g | - | 73 | >95 | 23 | Formstabil |
| 14 | PEG-PPG-Blockcopolymer 8000², 72 g | Vinylacetat, 410 g | - | 45 | >95 | 65 | Formstabil |

### Vergleichsbeispiele:

**Tabelle**

| Beispiel | Pfropfgrundlage (1) | Vinylester | Comonomer | K-Wert | Verseifungsgrad [%] | Reißdehnung [%] | Formstabilität der hergestellten Kapseln |
|---|---|---|---|---|---|---|---|
| 15 | PEG 20000, 273 g | Vinylacetat, 410 g | | 62 | 93 | 180 | Nicht Formstabil |
| 16 | PEG 35000 273 g | Vinylacetat, 410 g | | 59 | 94 | 404 | Nicht Formstabil |
| 17 | PEG 20000, 72 g | Vinylacetat, 410 g | | 60 | 93 | 141 | Nicht Formstabil |
| 18 | PEG 35000, 72 g | Vinylacetat, 410 g | | 65 | 94 | 280 | Nicht Formstabil |

| | | | | | | | |
|---|---|---|---|---|---|---|---|
| (1) PEG x: Polyethylenglykol mit mittlerem Molekulargewicht x | | | | | | | |

### Beispiel 19:

Man gibt bei Raumtemperatur unter Rühren innerhalb einer halben Stunde zu einer 19,3%igen wässerigen Lösung des Polymers aus Beispiel 16 eine 5%ige wässerige Lösung von Dinatriumtetraborat (Borax). Man beobachtet einen Viskositätsanstieg. Durch Zugabe der Borax-Lösung werden die aus den Polymerisaten hergestellten Kapseln formstabil.

| Menge an zugesetzter 5%iger Borax-Lösung [g] | Brookfieldviskosität (LVF, Spindel 2, 30 UpM, 23°C) [mPas] | Formstabilität der hergestellten Kapseln |
|---|---|---|
| 0 (entspricht Vergleichsbeispiel 16) | 110 | Nicht formstabil |
| 21,0 | 534 | Formstabil |
| 24,0 | 2228 | Formstabil |
| 26,9 | 7520¹ | Formstabil |
| 29,8 | 29190² | Formstabil |

| | | |
|---|---|---|
| ¹ Spindel 4, 30 UpM | | |
| ² Spindel 4, 6 UpM | | |

### Beispiel 20

Die Herstellung von Hartkapseln der Größe 0 erfolgte mittels des Tauchverfahrens. Beide Kapselhälften wurden getrennt hergestellt.

400 g Polymerisat von Vinylacetat und Polyethylenglykol 6000 im Verhältnis 95:5 wurde unter Rühren in 600g demineralisiertem Wasser bei 70°C gelöst. Dabei ist darauf zu achten, daß kein Schaum entsteht. In diese Lösung wurden am Ende abgerundete, mittels einer Silikonemulsion hydrophobierte Tauchstifte aus Edelstahl senkrecht eingetaucht, herausgezogen und unter ständigem Drehen um die eigene Achse in einem Warmluftstrom bei 90°C getrocknet. Die ständige Rotation ist wichtig, da es sonst zu Ungleichmäßigkeiten in der Wandstärke kommt. Die Tauchstifte für das Unterteil wiesen einen Durchmesser von 7,15 mm auf, die für das Oberteil einen Durchmesser von 7,45 mm. Nach der Trocknung wurden die Formkörper abgestreift, mit scharfen Messern auf die erforderliche Länge abgeschnitten und Oberteile und Unterteile zusammengesteckt.

Die Kapseln waren fest und zugleich elastisch. Nach 3monatiger Lagerung bei 23°C und 53% relativer Luftfeuchte waren keine Formveränderungen festzustellen.

### Vergleichsbeispiel

Die Herstellung von Hartkapseln der Größe 0 erfolgte mittels des Tauchverfahrens. Beide Kapselhälften wurden getrennt hergestellt.

400 g Polymerisat von Vinylacetat und Polyethylenglykol 20000 im Verhältnis 85:15 (Vergleichsbeispiel 17) wurde unter Rühren in 600g demineralisiertem Wasser bei 70°C gelöst. Dabei ist darauf zu achten, daß kein Schaum entsteht. In diese Lösung wurden am Ende abgerundete, mittels einer Silikonemulsion hydrophobierte Tauchstifte aus Edelstahl senkrecht eingetaucht, herausgezogen und unter ständigem Drehen um die eigene Achse in einem Warmluftstrom bei 90°C getrocknet. Die ständige Rotation ist wichtig, da es sonst zu Ungleichmäßigkeiten in der Wandstärke kommt. Die Tauchstifte für das Unterteil wiesen einen Durchmesser von 7,15 mm auf, die für das Oberteil einen Durchmesser von 7,45 mm. Nach der Trocknung wurden die Formkörper abgestreift, mit scharfen Messern auf die erforderliche Länge abgeschnitten und Oberteile und Unterteile zusammengesteckt.

Die Ober- und Unterteile ließen sich nur schwer von Edelstahlstiften lösen und ineinanderstecken. Die Kapseln waren sehr weich und verformbar. Nach 3monatiger Lagerung bei 23°C und 53 % relativer Luftfeuchte waren die Kapseln stark verformt, z.T. zu einem Polymerklumpen zusammengelaufen.

## Patentansprüche

1. Hartkapseln enthaltend
(A) Polymerisate, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren c)
und anschließender zumindest teilweiser Verseifung der Esterfunktionen der ursprünglichen Monomere a),
mit der Maßgabe, daß bei Abwesenheit eines weiteren copolymerisierbaren Monomers c), die polyetherhaltige Verbindung b) ein mittleres Molekulargewicht ≤ 10 000 (Zahlenmittel) haben muß,
(B) gegebenenfalls strukturverbessernde Hilfsstoffe und
(C) weitere übliche Bestandteile.

2. Hartkapseln gemäß Anspruch 1, **dadurch gekennzeichnet, daß** die Polymerisate (A) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I 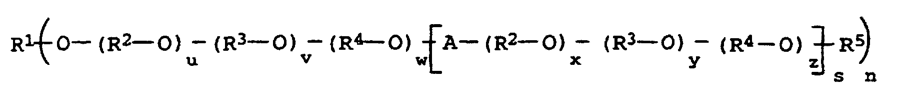 in der die Variablen unabhängig voneinander folgende Bedeutung haben:
R¹ Wasserstoff, C₁-C₂₄-Alkyl, R⁶-C(=O)-, R⁶-NH-C(=O)-, Polyalkoholrest;
R⁵ Wasserstoff, C₁-C₂₄-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂;
R⁶ C₁-C₂₄-Alkyl;
R⁷ Wasserstoff, C₁-C₂₄-Alkyl, R⁶-C(=O)-, R⁶-NH-C(=O)-;
A -C(=O)-O, -C(=O)-B-C(=O)-O, -C(=O)-NH-B-NH-C(=O)-O;
B -(CH₂)ₜ-, Arylen, ggf. substituiert;
n 1 bis 1000 (1-200);
s 0 bis 1000 (0-200);
t 1 bis 12 (1-12);
u 1 bis 5000 (1-250);
v 0 bis 5000 (0-250);
w 0 bis 5000 (0-250);
x 0 bis 5000 (0-250);
y 0 bis 5000 (0-250);
z 0 bis 5000 (0-250);
und
c) gegebenenfalls eines oder mehreren weiteren copolymerisierbaren Monomeren
und anschließender zumindest teilweiser Verseifung der Esterfunktionen der ursprünglichen Monomeren a), mit der Maßgabe, **daß** b) bei Abwesenheit von c) ein mittleres Molekulargewicht (Zahlenmittel) zwischen 300 und 10000 besitzt und n-z, die in Klammern angegebenen Werte haben.

3. Hartkapseln gemäß einem der Ansprüche 1 oder 2, **dadurch gekennzeichnet, daß** die Polymerisate (A) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 300 bis 100000 (nach dem Zahlenmittel), in der die Variablen unabhängig voneinander folgende Bedeutung haben:
R¹ Wasserstoff, C₁-C₁₂-Alkyl, R⁶-C(=O)-, R⁶-NH-C(=O)-, Polyalkoholrest;
R⁵ Wasserstoff, C₁-C₁₂-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂-;
R⁶ C₁-C₁₂ -Alkyl ;
R⁷ Wasserstoff, C₁-C₁₂-Alkyl, R⁶-C(=O)-, R⁶-NH-C(=O)-;
n 1 bis 8;
s 0;
u 2 bis 2000 (2-250);
v 0 bis 2000 (0-250);
w 0 bis 2000 (0-250);
und
c) gegebenenfalls eines oder mehreren weiteren copolymerisierbaren Monomeren
und anschließender zumindest teilweiser Verseifung der Esterfunktionen der ursprünglichen Monomeren a), mit der Maßgabe, **daß** b) bei Abwesenheit von c) ein mittleres Molekulargewicht (Zahlenmittel) zwischen 300 und 10000 besitzt und u-w, die in Klammern angegebenen Werte haben.

4. Hartkapseln gemäß einem der Ansprüche 1 bis 3, **dadurch gekennzeichnet, daß** die Polymerisate (A) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 500 bis 50000 (nach dem Zahlenmittel), in der die Variablen unabhängig voneinander folgende Bedeutung haben:
R¹ Wasserstoff, C₁-C₆-Alkyl, R⁶-C(=O)-, R⁶-NH-C(=O)-;
R⁵ Wasserstoff, C₁-C₆-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶ )-, -CH₂ -CHOR⁷-CH₂- ;
R⁶ C₁-C₆-Alkyl;
R⁷ Wasserstoff, C₁-C₆-Alkyl, R⁶-C(=O)-, R⁶-NH-C(=O)-;
n 1;
s 0;
u 5 bis 1000 (5-250);
v 0 bis 1000 (0-250);
w 0 bis 1000 (0-250);
und
c) gegebenenfalls eines oder mehreren weiteren copolymerisierbaren Monomeren
und anschließender zumindest teilweiser Verseifung der Esterfunktionen der ursprünglichen Monomeren a), mit der **Maßgabe, daß** b) bei Abwesenheit von c) ein mittleres Molekulargewicht (Zahlenmittel) zwischen 300 und 10000 besitzt und u-w, die in Klammern angegebenen Werte haben.

5. Hartkapseln gemäß einem der Ansprüche 1 bis 4, **dadurch gekennzeichnet, daß** die Polymerisate (A) erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls 0 bis 20 Gew.-% eines oder mehreren weiteren copolymerisierbaren Monomeren
und anschließender zumindest teilweiser Verseifung der Esterfunktionen der ursprünglichen Monomeren a), **dadurch gekennzeichnet, daß** die polyetherhaltigen Verbindungen b) durch Polymerisation von ethylenisch ungesättigten alkylenoxidhaltigen Monomeren und gegebenenfalls weiteren copolymerisierbaren Monomeren hergestellt worden sind.

6. Hartkapseln gemäß einem der Ansprüche 1 bis 5, **dadurch gekennzeichnet, daß** bei Anwesenheit mindestens eines weiteren copolymerisierbaren Monomers c), dieses ausgewählt wird aus der Gruppe:
Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie z.B. Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)-propyl]methacrylamid quaternisiert mit Methylchlorid, Methylsulfat oder Diethylsulfat.

7. Hartkapseln gemäß einem der Ansprüche 1 bis 8, **dadurch gekennzeichnet, daß** die Mengenverhältnisse
a) 10 bis 98 Gew.-%
b) 2 bis 90 Gew.-%
c) 0 bis 50 Gew.-%
betragen.

8. Hartkapseln gemäß einem der Ansprüche 1 bis 7, **dadurch gekennzeichnet, daß** die Mengenverhältnisse
a) 50 bis 97 Gew.-%
b) 3 bis 50 Gew.-%
c) 0 bis 20 Gew.-%
betragen.

9. Hartkapseln gemäß einem der Ansprüche 1 bis 8, **dadurch gekennzeichnet, daß** die Mengenverhältnisse
a) 65 bis 97 Gew.-%
b) 3 bis 35 Gew.-%
c) 0 bis 20 Gew.-%
betragen.

10. Hartkapseln gemäß einem der Ansprüche 1 bis 9, **dadurch gekennzeichnet, daß** die erhaltenen Polymerisate nachträglich durch eine polymeranaloge Umsetzung vernetzt werden.

11. Hartkapseln gemäß einem der Ansprüche 1 bis 10, **dadurch gekennzeichnet, daß** für die nachträgliche Vernetzung Dialdehyde, Diketone, Dicarbonsäuren, Borsäure, Borsäuresalze sowie Salze mehrwertiger Kationen eingesetzt werden.

12. Hartkapseln, gemäß einem der Ansprüche 1 bis 11, **dadurch gekennzeichnet, daß** als strukturverbessernde Hilfstoffe (B) folgende Verbindungsklassen eingesetzt werden:
a) Polymere mit einem Molekulargewicht größer 50000
b) Stoffe, die zu einer Vernetzung der Polymerketten der Polymere führen,
c) sowie gegebenenfalls Stoffen, die zu einer Vernetzung der Polymerketten der strukturverbessernden Hilfsstoffen **führen.**

13. Hartkapseln gemäß einem der Ansprüche 1-12, **dadurch gekennzeichnet, daß** als strukturverbessernde Hilfsstoffe Polymere aus folgenden Stoffklassen eingesetzt werden:
Polyaminosäuren, wie Gelatine, Zein, Sojaprotein sowie Derivate davon, Polysaccharide wie Stärke, abgebaute Stärke, Maltodextrine, Carboxymethylstärke, Cellulose, Hydroxypropylmethylcellulose, Hydroxypropylcellulose, Hydroxyethylcellulose, Methylcellulose, Carboxymethylcellulose, Ethylcellulose, Celluloseacetat, Celluloseacetatphthalat, Hydroxypropylcelluloseacetatphthalat, Hydroxypropylcelluloseacetatsuccinat, Hemicellulose, Galactomannane, Pectine, Alginate, Carrageenane, Xanthan, Gellan,Dextran,Curdlan, Pullulan, Gummi arabicum, Chitin, sowie Derivate davon, synthetische Polymere wie Polyacrylsäure, Polymethacrylsäure, Copolymerisate aus Acrylsäure- und Methacrylsäureestern, Polyvinylalkohole, Polyvinylacetat, Polyethylenglykole, Polyoxyethylen-Polyoxypropylen-Blockpolymere, Polyvinylpyrrolidone sowie Derivate davon.

14. Hartkapseln gemäß einem der Ansprüche 1 bis 13, **dadurch gekennzeichnet, daß** als weitere übliche Hüllbestandteile Füllstoffe, Formtrennmittel, Rieselhilfsmittel, Farbstoffe, Pigmente, Opakisierungsmittel, Aromen, Süßstoffe, Weichmacher, Konservierungsmittel und/oder Wirkstoffe enthalten sind.

15. Hartkapseln gemäß einem der Ansprüche 1 bis 14, **dadurch gekennzeichnet, daß** die Hülle besteht aus 10 bis 100 % Polymerisaten von Vinylestern auf Polyether gegebenenfalls 0 bis 80 % strukturverbessernden Hilfsstoffen und gegebenenfalls 0 bis 30 % weiteren üblichen Bestandteilen.

16. Hartkapseln gemäß einem der Ansprüche 1 bis 15, erhältlich durch das Tauchverfahren.

17. Hartkapseln gemäß einem der Ansprüche 1 bis 16, **dadurch gekennzeichnet, daß** sie einen oder mehrere pharmazeutische Wirkstoffe, Vitamine, Carotinoide, Mineralstoffe, Spurenelemente, Nahrungsergänzungsstoffe, kosmetische Wirkstoffe, Pflanzenschutzmittel, Badezusätze, Parfüm, Aroma, Reinigungsmittel oder Waschmittel beinhalten.

18. Hartkapseln gemäß einem der Ansprüche 1 bis 17, **dadurch gekennzeichnet, daß** in der Hülle 20 bis 80 %, eines magensaftresistenten Polymers enthalten sind.

19. Hartkapseln gemäß einem der Ansprüche 1 bis 18, **dadurch gekennzeichnet, daß** zur Erzielung einer Magensaftresistenz nach der Herstellung mit pharmazeutisch üblichen Coatingverfahren ein magensaftresistenter Überzug aufgebracht wird.

20. Verwendung der Hartkapseln gemäß einem der Ansprüche 1 bis 19, für pharmazeutische Anwendungen.

21. Verwendung der Hartkapseln gemäß einem der Ansprüche 1 bis 20 für kosmetische Anwendungen, Anwendungen im Pflanzenschutz, für Reinigungsmittel oder Nahrungsergänzungsmittel.

22. Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren copolymerisierbaren Monomeren
und anschließender zumindest teilweiser Verseifung der Esterfunktionen der ursprünglichen Monomeren a) mit der Maßgabe, daß b) bei Abwesenheit von c) ein mittleres Molekulargewicht (Zahlenmittel) zwischen 300 und 10.000 besitzt zur Herstellung von Hartkapseln gemäß einem der Ansprüche 1 bis 21.

## Claims

1. A hard capsule comprising
(A) polymers obtainable by free-radical polymerization of
a) at least one vinyl ester of C₁-C₂₄-carboxylic acids in the presence of
b) polyether-containing compounds and
c) where appropriate one or more other copolymerizable monomers c)
and subsequent at least partial hydrolysis of the ester functions in the original monomers a),
with the proviso that in the absence of another copolymerizable monomer c) the polyether-containing compound b) must have a number average molecular weight ≤10,000,
(B) where appropriate structure-improving auxiliaries and
(C) other conventional constituents.

2. A hard capsule as claimed in claim 1, wherein the polymers (A) are obtainable by free-radical polymerization of
a) at least one vinyl ester of C₁-C₂₄-carboxylic acids in the presence of
b) polyether-containing compounds of the general formula I 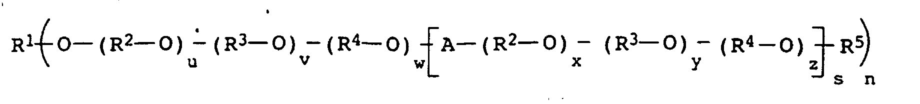 in which the variables have, independently of one another, the following meaning:
R¹ hydrogen, C₁-C₂₄-alkyl, R⁶-C(=O)-, R⁶-NH-C(=O)-, polyalcohol residue;
R⁵ hydrogen, C₁-C₂₄-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₂₄-alkyl;
R⁷ hydrogen, C₁-C₂₄-alkyl, R⁶-C(=O)-, R⁶-NH-C(=O)-;
A -C(=O)-O, -C(=O)-B-C(=O)-O, -C(=O)-NH-B-NH-C(=O)-O;
B -(CH₂)ₜ-, arylene, optionally substituted;
n 1 to 1000 (1-200);
s 0 to 1000 (0-200);
t 1 to 12 (1-12);
u 1 to 5000 (1-250);
v 0 to 5000 (0-250);
w 0 to 5000 (0-250);
x 0 to 5000 (0-250);
y 0 to 5000 (0-250);
z 0 to 5000 (0-250);
and
c) where appropriate one or more other copolymerizable monomers
and subsequent at least partial hydrolysis of the ester functions in the original monomers a), with the proviso that b) in the absence of c) has a number average molecular weight between 300 and 10,000, and n-z have the values stated in parentheses.

3. A hard capsule as claimed in either of claims 1 or 2, wherein the polymers (A) are obtainable by free-radical polymerization of
a) at least one vinyl ester of C₁-C₂₄-carboxylic acids in the presence of
b) polyether-containing compounds of the general formula I with a number average molecular weight of from 300 to 100,000, in which the variables have, independently of one another, the following meaning:
R¹ hydrogen, C₁-C₁₂-alkyl, R⁶-C(=O)-, R⁶-NH-C(=O)-, polyalcohol residue;
R⁵ hydrogen, C₁-C₁₂-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₁₂-alkyl;
R⁷ hydrogen, C₁-C₁₂-alkyl, R⁶-C(=O)-, R⁶-NH-C(=O)-;
n 1 to 8;
s 0;
u 2 to 2000 (2-250);
v 0 to 2000 (0-250);
w 0 to 2000 (0-250);
and
c) where appropriate one or more other copolymerizable monomers
and subsequent at least partial hydrolysis of the ester functions in the original monomers a), with the proviso that b) in the absence of c) has a number average molecular weight between 300 and 10,000, and u-w have the values stated in parentheses.

4. A hard capsule as claimed in any of claims 1 to 3, wherein the polymers (A) are obtainable by free-radical polymerization of
a) at least one vinyl ester of C₁-C₂₄-carboxylic acids in the presence of
b) polyether-containing compounds of the general formula I with a number average molecular weight of from 500 to 50,000, in which the variables have, independently of one another, the following meaning:
R¹ hydrogen, C₁-C₆-alkyl, R⁶-C(=O)-, R⁶-NH-C(=O)-;
R⁵ hydrogen, C₁-C₆-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₆-alkyl;
R⁷ hydrogen, C₁-C₆-alkyl, R⁶-C(=O)-, R⁶-NH-C(=O)-;
n 1;
s 0;
u 5 to 1000 (5-250);
v 0 to 1000 (0-250);
w 0 to 1000 (0-250);
and
c) where appropriate one or more other copolymerizable monomers
and subsequent at least partial hydrolysis of the,ester functions in the original monomers a), with the proviso that b) in the absence of c) has a number average molecular weight between 300 and 10,000, and u-w have the values stated in parentheses.

5. A hard capsule as claimed in any of claims 1 to 4, wherein the polymers (A) are obtainable by free-radical polymerization of
a) at least one vinyl ester of C₁-C₂₄-carboxylic acids in the presence of
b) polyether-containing compounds and
c) where appropriate 0 to 20% by weight of one or more other copolymerizable monomers
and subsequent at least partial hydrolysis of the ester functions in the original monomers a), wherein the polyether-containing compounds b) have been prepared by polymerization of ethylenically unsaturated alkylene oxide-containing monomers, and, where appropriate, other copolymerizable monomers.

6. A hard capsule as claimed in any of claims 1 to 5, wherein if at least one other copolymerizable monomer c) is present it is selected from the group:
acrylic acid, methacrylic acid, maleic acid, fumaric acid, crotonic acid, maleic anhydride and its monoesters, methyl acrylate, methyl methacrylate, ethyl acrylate, ethyl methacrylate, n-butyl acrylate, n-butyl methacrylate, t-butyl acrylate, t-butyl methacrylate, isobutyl acrylate, isobutyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylates, 2-hydroxyethyl methacrylate, hydroxypropyl methacrylates, alkylene glycol (meth)acrylates, styrene, unsaturated sulfonic acids such as, for example, acrylamidopropanesulfonic acid, vinylpyrrolidone, vinylcaprolactam, vinyl ethers (for example: methyl, ethyl, butyl or dodecyl vinyl ether), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methyl sulfate, N,N-dimethylaminoethyl methacrylate, N-[3-(dimethylamino)propyl]methacrylamide quaternized with methyl chloride, methyl sulfate or diethyl sulfate.

7. A hard capsule as claimed in any of claims 1 to 6, wherein the ratios of amounts are
a) 10 to 98% by weight
b) 2 to 90% by weight
c) 0 to 50% by weight.

8. A hard capsule as claimed in any of claims 1 to 7, wherein the ratios of amounts are
a) 50 to 97% by weight
b) 3 to 50% by weight
c) 0 to 20% by weight.

9. A hard capsule as claimed in any of claims 1 to 8, wherein the ratios of amounts are
a) 65 to 97% by weight
b) 3 to 35% by weight
c) 0 to 20% by weight.

10. A hard capsule as claimed in any of claims 1 to 9, wherein the resulting polymers are subsequently crosslinked by a polymer-analogous reaction.

11. A hard capsule as claimed in any of claims 1 to 10, wherein dialdehydes, diketones, dicarboxylic acids, boric acid, boric acid salts, and salts of multiply charged cations are employed for the subsequent crosslinking.

12. A hard capsule as claimed in any of claims 1 to 11, wherein the structure-improving auxiliaries (B) employed are the following classes of compounds:
a) polymers with a molecular weight greater than 50,000
b) substances which lead to crosslinking of the polymer chains of the polymers,
c) and, where appropriate, substances which lead to crosslinking of the polymer chains of the structure-improving auxiliaries.

13. A hard capsule as claimed in any of claims 1-12, wherein the structure-improving auxiliaries employed are polymers from the following classes of substances:
polyamino acids such as gelatin, zein, soybean protein and derivatives thereof, polysaccharides such as starch, degraded starch, maltodextrins, carboxymethylstarch, cellulose, hydroxypropylmethylcellulose, hydroxypropylcellulose, hydroxyethylcellulose, methylcellulose, carboxymethylcellulose, ethylcellulose, cellulose acetate, cellulose acetate phthalate, hydroxypropylcellulose acetate phthalate, hydroxypropylcellulose acetate succinate, hemicellulose, galactomannans, pectins, alginates, carrageenans, xanthan, gellan, dextran, curdlan, pullulan, gum arabic, chitin, and derivatives thereof, synthetic polymers such as polyacrylic acid, polymethacrylic acid, copolymers of acrylic esters and methacrylic esters, polyvinyl alcohols, polyvinyl acetate, polyethylene glycols, polyoxyethylene/polyoxypropylene block copolymers, polyvinylpyrrolidones and derivatives thereof.

14. A hard capsule as claimed in any of claims 1 to 13, which comprises as other conventional shell constituents fillers, release agents, flow aids, dyes, pigments, opacifiers, flavorings, sweeteners, plasticizers, preservatives and/or active ingredients.

15. A hard capsule as claimed in any of claims 1 to 14, wherein the shell consists of 10 to 100% polymers of vinyl esters on polyether, where appropriate 0 to 80% structure-improving auxiliaries and, where appropriate, 0 to 30% other conventional constituents.

16. A hard capsule according to any of claims 1 to 15, obtainable by the dip process.

17. A hard capsule as claimed in any of claims 1 to 16, which comprises one or more active pharmaceutical ingredients, vitamins, carotenoids, minerals, trace elements, food supplements, cosmetic active ingredients, crop protection agents, bath additives, perfume, flavoring, cleaner or detergent.

18. A hard capsule as claimed in any of claims 1 to 17, wherein the shell comprises from 20 to 80% of a polymer resistant to gastric fluid.

19. A hard capsule as claimed in any of claims 1 to 18, wherein a coating resistant to gastric fluid is applied using pharmaceutically customary coating processes to achieve resistance to gastric fluid after production.

20. The use of the hard capsule as claimed in any of claims 1 to 19 for pharmaceutical applications.

21. The use of the hard capsule as claimed in any of claims 1 to 19 for cosmetic applications, applications in crop protection, or for cleaners or food supplements.

22. The use of polymers obtainable by free-radical polymerization of
a) at least one vinyl ester of C₁-C₂₄-carboxylic acids in the presence of
b) polyether-containing compounds and
c) where appropriate one or more copolymerizable monomers
and subsequent at least partial hydrolysis of the ester functions in the original monomers a), with the proviso that b) in the absence of c) has a number average molecular weight between 300 and 10,000 for producing a hard capsule as claimed in any of claims 1 to 19.

## Revendications

1. Capsules dures contenant
(A) des polymères qui peuvent être obtenus par polymérisation radicalaire de
a) au moins un ester vinylique d'acides carboxyliques en C₁-C₂₄ en présence de
b) des composés contenant des polyéthers et
c) éventuellement un ou plusieurs autres monomères copolymérisables c)
et saponification subséquente au moins partielle des fonctions ester des monomères a) initiaux,
avec pour condition que, en l'absence d'un autre monomère c) copolymérisable, le composé b) contenant des polyéthers doit avoir une masse moléculaire moyenne ≤ 10 000 (moyenne en nombre),
(B) éventuellement des adjuvants d'amélioration de structure et
(C) d'autres composants classiques.

2. Capsules dures selon la revendication 1, **caractérisées par le fait que** les polymères (A) peuvent être obtenus par polymérisation radicalaire de
a) au moins un ester vinylique d'acides carboxyliques en C₁-C₂₄ en présence de
b) des composés contenant des polyéthers de formule générale I 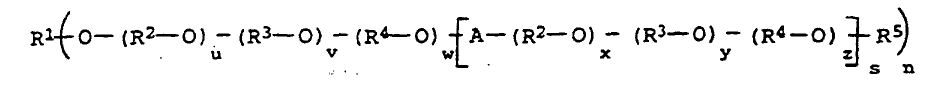 dans laquelle les variables ont, indépendamment l'une de l'autre, la signification suivante:
R¹ hydrogène, alkyle en C₁-C₂₄, R⁶-C(=O)-, R⁶-NH-C(=O)-, un reste polyalcool;
R5 hydrogène, alkyle en C₁-C₂₄, R⁶-C(=O)-, R⁶-NH-C(=O)-;
R² à R⁴ -(CH₂)₂-, -(CH₂)₃-, -(CH₂)₄-, -CH₂-CH(R⁶)-, -CH₂-CHOR⁷-CH₂-;
R⁶ alkyle en C₁-C₂₄;
R⁷ hydrogène, alkyle en C₁-C₂₄, R⁶-C(=O)-, R⁶-NH-C(=O)-;
A -C(=O)-O, -C(=O)-B-C(=O)-O, -C(=O)-NH-B-NH-C(=O)-O;
B -(CH₂)ₜ-, arylène, éventuellement substitué;
n 1 à 1000 (1-200);
s 0 à 1000 (0-200);
t 1 à 12 (1-12);
u 1 à 5000 (1-250);
v 0 à 5000 (0-250);
w 0 à 5000 (0-250);
x 0 à 5000 (0-250);
y 0 à 5000 (0-250);
z 0 à 5000 (0-250);
et
c) éventuellement un ou plusieurs autres monomères copolymérisables
et subséquemment saponification au moins partielle des fonctions ester des monomères initiaux a), avec pour condition que b) en l'absence de c) possède une masse moléculaire moyenne (moyenne en nombre) entre 300 et 10000, et n-z ont les valeurs indiquées entre parenthèses.

3. Capsules dures selon l'une des revendications 1 ou 2, **caractérisées par le fait que** les polymères (A) peuvent être obtenus par polymérisation radicalaire de
a) au moins un ester vinylique d'acides carboxyliques en C₁-C₂₄ en présence de
b) des composés contenant des polyéthers de formule générale I ayant une masse moléculaire moyenne de 300 à 10000 (selon la moyenne en nombre), dans laquelle les variables ont, indépendamment l'une de l'autre, la signification suivante :
R¹ hydrogène, alkyle en C₁-C₁₂, R⁶-C(=O)-, R⁶-NH-C(=O)-, un reste polyalcool;
R5 hydrogène, alkyle en C₁-C₂₂, R⁶-C(=O)-, R⁶-NH-C(=O)-;
R² à R⁴ -(CH₂)₂-, -(CH₂)₃-, -(CH₂)₄-, -CH₂-CH(R⁶)-, -CH₂-CHOR⁷-CH₂-;
R⁶ alkyle en C₁-C₁₂;
R⁷ hydrogène, alkyle en C₁-C₁₂, R⁶-C(=O)-, R⁶-NH-C(=O)-;
n 1 à 8;
s 0;
u 2 à 2000 (2-250);
v 0 à 2000 (0-250);
w 0 à 2000 (0-250);
et
c) éventuellement un ou plusieurs autres monomères copolymérisables
et subséquemment saponification au moins partielle des fonctions ester des monomères initiaux a), avec pour condition que b) en l'absence de c) possède une masse moléculaire moyenne (moyenne en nombre) entre 300 et 10000, et u-w ont les valeurs indiquées entre parenthèses.

4. Capsules dures selon l'une des revendications 1 à 3, **caractérisées par le fait que** les polymères (A) peuvent être obtenus par polymérisation radicalaire de
a) au moins un ester vinylique d'acides carboxyliques en C₁-C₂₄ en présence de
b) des composés contenant des polyéthers de formule générale I ayant une masse moléculaire moyenne de 500 à 50000 (selon la moyenne en nombre), dans laquelle les variables ont, indépendamment l'une de l'autre, la signification suivante :
R¹ hydrogène, alkyle en C₁-C₆, R⁶-C(=O)-, R⁶-NH-C(=O)-;
R5 hydrogène, alkyle en C₁-C₆, R⁶-C(=O)-, R⁶-NH-C(=O)-;
R² à R⁴ -(CH₂)₂-, -(CH₂)₃-, -(CH₂)₄-, -CH₂-CH(R⁶)-, -CH₂-CHOR⁷-CH₂-;
R⁶ alkyle en C₁-C₆;
R⁷ hydrogène, alkyle en C₁-C₆, R⁶-C(=O)-, R⁶-NH-C(=O)-;
n 1;
s 0;
u 5 à 1000 (5-250);
v 0 à 1000 (0-250);
w 0 à 1000 (0-250);
et
c) éventuellement un ou plusieurs autres monomères copolymérisables
et subséquemment saponification au moins partielle des fonctions ester des monomères initiaux a), avec pour condition que b) en l'absence de c) possède une masse moléculaire moyenne (moyenne en nombre) entre 300 et 10000, et u-w ont les valeurs indiquées entre parenthèses.

5. Capsules dures selon l'une des revendications 1 à 4, **caractérisées par le fait que** les polymères (A) peuvent être obtenus par polymérisation radicalaire de
a) au moins un ester vinylique d'acides carboxyliques en C₁-C₂₄ en présence de
b) des composés contenant des polyéthers et
c) éventuellement 0 à 20 % en poids d'un ou plusieurs autres monomères copolymérisables
et saponification subséquente au moins partielle des fonctions ester des monomères initiaux a), tandis que les composés contenant des polyéther b) ont été préparés par polymérisation de monomères contenant de l'oxyde d'alkylène et à insaturation éthylénique, et éventuellement d'autres monomères copolymérisables.

6. Capsules dures selon l'une des revendications 1 à 5, **caractérisées par le fait que**, au moins un autre monomère copolymérisable c) étant présent, celui-ci est choisi dans le groupe:
acide acrylique, acide méthacrylique, acide maléique, acide fumarique, acide crotonique, anhydride d'acide maléique, ainsi que leurs hémi-esters, acrylate de méthyle, méthacrylate de méthyle, acrylate d'éthyle, méthacrylate d'éthyle, acrylate de n-butyle, méthacrylate de n-butyle, acrylate de tert-butyle, méthacrylate de tert-butyle, acrylate d'isobutyle, méthacrylate d'isobutyle, acrylate de 2-éthylhexyle, acrylate de stéaryle, méthacrylate de stéaryle, N-tert-butylacrylamide, N-octylacrylamide, acrylate de 2-hydroxyéthyle, acrylates d'hydroxypropyle, méthacrylate de 2-hydroxyéthyle, méthacrylates d'hydroxypropyle, (méth)acrylates d'alkylèneglycol, styrène, acides sulfoniques insaturés tels que par exemple acide acrylamidopropanesulfonique, pyrrolidone de vinyle, vinyl-caprolactame, éther vinylique (par exemple: éther de méthyle, d'éthyle, de butyle ou de dodécyle et de vinyle), vinylformamide, vinylméthylacétamide, vinylamine, 1-vinylimidazole, 1-vinyl-2-méthylimidazole, méthacrylate de N,N-diméthylaminométhyle et N-[3-(diméthylamino)propyl]méthacrylamide; chlorure de 3-méthyl-1-vinylimidazolium, méthylsulfate de 3-méthyl-1-vinylimidazolium, méthacrylate de N,N-diméthylaminoéthyle, N-[3-(diméthylamino)-propyl]méthacrylamide quaternisé avec du chlorure de méthyle, du sulfate de méthyle ou du sulfate de diéthyle.

7. Capsules dures selon l'une des revendications 1 à 8, **caractérisées par le fait que** les proportions valent
a) 10 à 98 % en poids
b) 2 à 90 % en poids
c) 0 à 50 % en poids.

8. Capsules dures selon l'une des revendications 1 à 7, **caractérisées par le fait que** les proportions valent
a) 50 à 97 % en poids
b) 3 à 50 % en poids
c) 0 à 20 % en poids.

9. Capsules dures selon l'une des revendications 1 à 8, **caractérisées par le fait que** les proportions valent
a) 65 à 97 % en poids
b) 3 à 35 % en poids
c) 0 à 20 % en poids.

10. Capsules dures selon l'une des revendications 1 à 9, **caractérisées par le fait que** les polymères obtenus sont ultérieurement réticulés par une réaction de même type de polymérisation.

11. Capsules dures selon l'une des revendications 1 à 9, **caractérisées par le fait qu'**on utilise pour la réticulation ultérieure des dialdéhydes, des dicétones, des acides dicarboxyliques, de l'acide borique, des sels d'acide borique, ainsi que des sels de cations plurivalents.

12. Capsules dures selon l'une des revendications 1 à 11, **caractérisées par le fait qu'**on utilise comme adjuvants d'amélioration de structure (B) les classes de composés suivantes:
a) polymères ayant une masse moléculaire supérieure à 50000
b) substances qui conduisent à une réticulation des chaînes polymères des polymères,
c) ainsi que, éventuellement, substances qui conduisent à une réticulation des chaînes polymères des adjuvants d'amélioration de structure.

13. Capsules dures selon l'une des revendications 1-12, **caractérisées par le fait qu'**on utilise comme adjuvants d'amélioration de structure des polymères des classes de substances suivantes:
poly(acides aminés), tels que gélatine, zéine, protéine de soja, ainsi que leurs dérivés, polysaccharides tels que amidon, amidon dégradé, maltodextrine, ccarboxyméthylamidon, cellulose, hydroxypropyl-méthylcellulose, hydroxypropylcellulose, hydroxyéthylcellulose, méthylcellulose, carboxyméthylcellulose, éthylcellulose, acétate de cellulose, acétophtalate de cellulose, acétophtalate d'hydroxypropyl-cellulose, acétosuccinate d'hydroxypropylcellulose, hémi-cellulose, galactomannane, pectine, alginate, carraghénane, xanthane, gellane, dextrane, curdlane, pullulane, gomme arabique, chitine, ainsi que leurs dérivés, polymères synthétiques tels que poly(acide acrylique), poly(acide méthacrylique), copolymères d'esters d'acide acrylique et d'acide méthacrylique, poly(alcools vinyliques), poly(acétate de vinyle), polyéthylèneglycols, copolymères séquencés de polyoxyéthylène-polyoxypropylène, poly(pyrrolidone de vinyle, ainsi que leurs dérivés.

14. Capsules dures selon l'une des revendications 1 à 13, **caractérisées par le fait qu'**elles contiennent comme autres constituants classiques de l'enveloppe: des charges, des agents de démoulage, des adjuvants d'écoulement, des colorants, des pigments, des agents d'opacification, des arômes, des édulcorants, des plastifiants, des conservateurs et/ ou des substances actives.

15. Capsules dures selon l'une des revendications 1 à 14, **caractérisées par le fait que** l'enveloppe consiste en 10 à 100 % de polymères d'esters vinyliques sur des polyéthers, éventuellement 0 à 80 % d'adjuvants d'amélioration de structure et éventuellement 0 à 30 % d'autres constituants classiques.

16. Capsules dures selon l'une des revendications 1 à 15, pouvant être obtenues par le procédé par immersion.

17. Capsules dures selon l'une des revendications 1 à 16, **caractérisées par le fait qu'**elles comportent une ou plusieurs substances à activité pharmaceutique, des vitamines, des caroténoïdes, des minéraux, des oligo-éléments, des compléments alimentaires, des substances à activité cosmétique, des agents phytosanitaires, des additifs pour le bain, des parfums, des arômes, des agents de nettoyage ou des agents de lavage.

18. Capsules dures selon l'une des revendications 1 à 17, **caractérisées par le fait que** sont contenus dans l'enveloppe 20 à 80 % d'un polymère résistant au suc gastrique.

19. Capsules dures selon l'une des revendications 1 à 18, **caractérisées par le fait qu'**on applique, pour obtenir une résistance au suc gastrique, un revêtement résistant au suc gastrique, après la préparation avec le procédé de revêtement classique dans l'industrie pharmaceutique.

20. Utilisation des capsules dures selon l'une des revendications 1 à 19, pour des applications pharmaceutiques.

21. Utilisation des capsules dures selon l'une des revendications 1 à 20 pour des applications cosmétiques, des applications dans le secteur phytosanitaire, pour le nettoyage ou pour des compléments alimentaires.

22. Utilisation de polymères qui peuvent être obtenus par polymérisation radicalaire de
a) au moins un ester vinylique d'acides carboxyliques en C₁-C₂₄ en présence de
b) des composés contenant des polyéthers et
c) éventuellement un ou plusieurs monomères copolymérisables,
et saponification subséquente au moins partielle des fonctions ester des monomères initiaux a), avec pour condition que b) en l'absence de c) possède une masse moléculaire moyenne (moyenne en nombre) entre 300 et 10.000, pour la préparation de capsules dures selon l'une des revendications 1 à 21.
